# EUROPEAN PATENT APPLICATION

(11) **EP 4 600 270 A1**
(43) Date of publication of application: **13.08.2025**
(21) Application number: 24156565.4
(22) Date of filing: 08.02.2024
(51) Int. Cl.: C07K 16/28, A61K 51/00, G01N 33/574

(54) **ANTI-CD7 SINGLE DOMAIN ANTIBODY (SDAB), A PHARMACEUTICAL COMPOSITION AND A KIT FOR USE IN THE DIAGNOSIS AND/OR THERAPY OF CANCER**

(71) Applicant: ImnoTech GmbH, 81675 München (DE)
(72) Inventor: Krackhardt, Prof. Dr. Angela M., 24939 Flensburg (DE); Weber, Prof. Dr. Wolfgang A., 81675 München (DE); Russelli, Dr. Lisa, 81675 München (DE); D'Alessandria, PD Dr. Calogero, 81675 München (DE); Gosmann, Dr. Dario, 81675 München (DE); Mayer, Dr. Kristine, 81675 München (DE)
(74) Representative: Fuchs Patentanwälte Partnerschaft mbB

(57) **Abstract**

The present invention relates a sdAb which binds to human CD7 for use in the diagnosis and/or therapy of cancer, preferably comprising at least one of a detectable label, a chelator, a prosthetic group, a therapeutically and/or pharmacologically active agent, a pharmaceutical composition comprising the sdAb and a kit.

## Description

The present invention relates a sdAb which binds to human CD7 for use in the diagnosis and/or therapy of cancer, preferably comprising at least one of a detectable label, a chelator, a prosthetic group, a therapeutically and/or pharmacologically active agent, a pharmaceutical composition comprising the sdAb and a kit.

Immunotherapeutic approaches have proven to be very effective in the treatment of various tumor diseases. In particular, immune checkpoint inhibitors such as anti-PD-1 and anti-CTLA-4 have already been authorised for the therapy of many tumor entities. However, also possibilities of adoptive cell transfer of genetically modified T cells, i.e. the use of chimeric antigen receptor T cells (CAR T cells) or T cell receptor (TCR)-transduced T cells, showed first promising clinical results, which is reflected in the European-wide approval of two CD19-specific CAR T cells by the EMA (European Medicines Agency) (Druker et al. 2006).

With increasing integration of these new therapeutic options into trials and clinical practice, adequate monitoring of individual therapy progress is becoming increasingly important.

Established imaging modalities such as computed tomography (CT), magnetic resonance imaging (MRI), and functional imaging modalities such as positron emission tomography (PET) have high spatial and temporal resolution but are limited in their application for monitoring the progress of immunotherapies (Lang et al. 2020). This results in poor differentiation between progression and pseudoprogression of tumors, i.e., increase in size of lesions due to tumor growth or immigration of immune cells (Dromain et al. 2020).

The 2009 revision of the RECIST (Response Evaluation Criteria in Solid Tumors) guidelines specifically for immunotherapies (irRECIST) represents a first attempt to improve the assessment of therapy progression, but there is still a lack of an appropriate, sensitive, clinically applicable imaging modality. It would be extremely important to be able to evaluate the response to therapy at an early stage in order to avoid premature discontinuation and unnecessary continuation of therapy, as well as to promptly detect T-cell infiltration in healthy tissue as an expression of an autoimmune event. Furthermore, adequate monitoring would significantly deepen the current understanding of pharmacokinetics and dynamics of immunotherapies.

A promising imaging approach is the direct visualization of T cells, which play the key role in immunotherapies. Weist et al. describes an approach, wherein CAR T cells and TCR-transduced T cells are radiolabeled ex vivo or equipped with a reporter gene prior to adoptive cell transfer (Weist et al. 2018). However, this has the disadvantage that in the case of radioactive labeling, these can only be imaged over a short period of time, while, for example, the effects of immune checkpoint inhibitors on endogenous T cells cannot be assessed. Furthermore disadvantageously, functional impairment of immune cells was often shown (Auletta et al. 2018).

Alternatively, immuno-PET is currently examined for the feasibility of detection of T cells. Here, antibodies or their derivatives directed against specific surface antigens of T cells are radioactively labeled, allowing T cells to be imaged at any time point in vivo. The selection of a suitable target antigen, which is highly specific for T cells, as well as an antibody derivative, which does not affect the functionality of the T cells when the antigen is bound, is decisive.

WO 2021/113 450 A2 describes a non-invasive imaging methods for diagnosis, prediction, prognosis, and treatment of a disease, in particularselected from a solid tumor, a non-solid tumor, an autoimmune disease, an infectious disease (including without limitation, viral, bacterial, or fungal infections), comprising administering to a subject a first antigen-binding construct comprising a first radionuclide tracer, wherein the antigen-binding construct selectively binds a first target selected from CD3, CD4, and CD8, and estimating a distribution and/or abundance of cells expressing the first target in one or more tissues of the subject using positron emission tomography (PET) or single photon emission computed tomography (SPECT) to measure a level of the first radionuclide tracer in the subject. Preferably, the antigen-binding construct is a Fab', F(ab')₂, Fv, rlgG (reduced IgG), a scFv fragment, a minibody, a diabody, a cys-diabody, or a sdAb.

Mayer et al. describe CD7 as target for monitoring T cells in the context of immunotherapies (Mayer et al. 2018). T cells incubated with anti-CD2 and anti-CD7 F(ab')₂ showed no major modulation of functionality in vitro, and PET imaging provided a distinct and strong signal at the tumor site using the respective zirconium-89-labeled radiotracers. T-cell tracking by anti-CD7 F(ab')₂ had no long-term impact on T-cell functionality in vivo and anti-CD7 F(ab')₂ imaging did not alter tumor rejection. Additionally, Mayer et al. have shown that CD7 is slightly upregulated on specifically activated T cells and present on both CD4+ and CD8+ T cells.

US 10 301 389 B2 describes antigen-binding constructs, in particular cys-diabodies or minibodies, to CD3 and the use for modulating the biologic activity associated with CD3 expression on immune cells, for targeting therapeutic agents to cells that express the CD3 protein, and for detecting the presence or absence of CD3.

US 2021/0371527 A1 describes antigen binding constructs, in particular cys-diabodies or minibodies, binding to CD4 and the use for detection, diagnosis, surgery, staging, treatment, monitoring of treatment, monitoring of disease progression, and monitoring therapy.

US 10 414 820 B2 describes cys-diabodies or minibodies binding to CD8 and the use for the detection of human CD8, in particular for diagnostic imaging of the immune system, preferably in vivo detection of T-cell localization.

US 2017/0226204 A1 discloses nanometer antibodies for human CD7 molecule and an encoding DNA sequence thereof. The nanometer antibody is expressed in Escherichia coli, used for preparing an agent for the detection of CD7 molecule and targeted therapy, and for flow cytometry and cellular immunofluorescence assays.

Furthermore, Käsbauer et al. and Tang et al. describe anti-CD7 sdAbs for flow cytometry and cellular immunofluorescence assays (Käsbauer et al. 2019, Tang et al. 2016)

The object is therefore to provide an alternative marker for the diagnosis and/or therapy of cancer related to the T cell binding.

According to the invention, the object is solved by the sdAb which binds to human CD7 for use in the diagnosis and/or therapy of cancer, the nucleic acid, the pharmaceutical composition, and the kit according to the independent claims. Advantageous embodiments of the invention are indicated in the dependent claims.

A first aspect of the invention provides a sdAb which binds to human CD7 which binds to human CD7 comprising complementarity determining regions (CDR): CDR1 with an amino acid sequence selected from the group comprising SEQ ID No. 14 to SEQ ID No. 17, CDR2 with an amino acid sequence selected from the group comprising SEQ ID No. 18 to SEQ ID No. 20 and CDR3 with an amino acid sequence selected from the group comprising SEQ ID No. 21 to SEQ ID No. 25, for use in the diagnosis and/or therapy of cancer in vivo.

As used herein, the term "sdAb" (single domain antibody) refers to an antibody fragment consisting of a single monomeric variable antibody domain and selectively binds to a specific antigen, in particular CD7. Advantageously, the sdAbs according to the invention have a small size and good binding properties. The sdAb can circulate rapidly through the bloodstream, penetrate the target tissue and bind to immune cells, but is also rapidly excreted and not perceived as a harmful substance in a patient, and does not affect any physiological parameters of a patient.

As used herein, the term "CD7" (cluster of differentiation 7) refers to a cell adhesion molecule found on the surface of T cells, natural killer (NK) cells and thymocytes, preferably human CD7 according to SEQ ID No. 1.

As used herein, the term "cancer" refers to a disease involving abnormal cell growth with the potential to invade or spread to other parts of the body.

In embodiments, the sdAb which binds to human CD7 (anti-CD7-sdAbs) according to the invention is used for the diagnosis of cancer by the direct imaging of immune cells, in particular T cells, NK cells, thymocytes or dendritic cells, preferably T cells, in immunotherapies.

Advantageously, response patterns of cancer immunotherapies can be evaluated using thesdAb which binds to human CD7. Advantageously, CD7 occurs on each subgroup of the immune cells and thus each kind of immunotherapy can be evaluated. Further advantageously, CD7 occurs on activated immune cells at a slightly higher density and thus, the tumor treating immune cells can be imaged more clearly.

Further advantageously, the binding of the sdAb to the target molecule CD7 does not affect the function of the immune cells, in particular T cell and/or NK cells.

In further embodiments, the sdAb which binds to human CD7 according to the invention is used for theranostics, i.e. the combined diagnosis and therapy, of immune cell related cancer, preferably lymphomas, in particular T cell or NK lymphomas; or leukaemia in vivo.

As used herein, the term "lymphoma" refers to "malignant lymphomas", which are cancers of the lymphatic system, including the lymph nodes, tonsils and spleen, and lymphatic tissue in the stomach, intestines or skin. In lymphoma, white blood cells, the so-called lymphocytes, which belong to the lymphatic system, grow uncontrollably.

As used herein, the term "leukaemia" refers to a group of blood cancers with a high number of abnormal white blood cells.

In embodiments, the diagnosis is carried out before, during and/or after immunotherapy of cancer. In embodiments, the sdAb is used as radioactive tracer and/or contrast agent in non-invasive medical imaging of T cells in vivo, preferably by PET or SPECT imaging; or for the synthesis of radioactive tracers and/or contrast agents ex vivo.

Advantageously, the use of a sdAb which binds to human CD7 for diagnosis of cancer during therapy allows precise observation of whether immune cells are present on and in the tumor, which in turn indicates whether the tumor is being fought and the immunotherapy is effective. Additionally, if there are no immune cells on the tumor, this can also be determined promptly and the patients can be switched to a different therapy. Thus, patients gain enormously valuable time and at the same time an extremely costly immunotherapy without benefit does not have to be administered over several weeks. The time gained for patients is essential, because while CT or MRI can only detect tumor growth or tumor regression after up to two months, infiltration of the tumor with T cells can be made visible after only 1 to 2 weeks. Accordingly, important therapy decisions can be made much earlier.

Furthermore, different tumor types are no limitation, since the sdAb which binds to human CD7 does not visualize the tumor itself, but the immune cells used for immunotherapy. Purposefully, the activated T cells, i.e. those that actively fight the tumor, are bound. The increased density of the target molecules (CD7) also allows more tracer to bind to the T cells, which in turn allows just the tumor-fighting cells to be visualized more clearly.

In embodiments, the sdAb comprises an amino acid sequence according to one of the sequences selected from SEQ ID No. 2 to SEQ ID No. 13.

In embodiments, the sdAb comprises an amino acid sequence with at least 90% sequence similarity with one of the sequences selected from SEQ ID No. 2 to SEQ ID No. 13, preferably an amino acid sequence with at least 95% sequence similarity with one of the sequences selected from SEQ ID No. 2 to SEQ ID No. 13, most preferably an amino acid sequence with at least 99% sequence similarity with one of the sequences selected from SEQ ID No. 2 to SEQ ID No. 13. As used herein, the term "similarity" refers to the sequence homology, wherein conservative substitutions of amino acid residues having similar physicochemical properties over the length of the amino acid sequence are comprised. The % sequence similarity is determined with any reasonable similarity-scoring matrix known by the person skilled in the art, preferably with a similarity-scoring matrix selected from BLOSUM50, BLOSUM62, PMBEC orVTML10 to VTML80. In embodiments, the sdAb comprises an amino acid sequence with at least 90%, preferably at least 95%, more preferably at least 99%; sequence identity with one of the sequences selected from SEQ ID No. 2 to SEQ ID No. 13. As used herein, the term "% sequence identity" refers to the number of identical amino acid residues in relation to the length of the amino acid sequence.

In embodiments, the sdAb comprises an amino acid sequence according to one of the sequences selected from SEQ ID No. 2, SEQ ID No. 4, SEQ ID No. 7, SEQ ID No. 8, SEQ ID No. 9 and SEQ ID No. 13, preferably an amino acid sequence according to one of the sequences selected from SEQ ID No. 2, SEQ ID No. 4, SEQ ID No. 7, SEQ ID No. 8 and SEQ ID No. 9.

In embodiments, the sdAb comprises an amino acid sequence according to one of the sequences selected from SEQ ID No. 2, SEQ ID No. 6, SEQ ID No. 10 and SEQ ID No. 11, preferably an amino acid sequence according to one of the sequences selected from SEQ ID No. 2, SEQ ID No. 10 and SEQ ID No. 11.

In embodiments, the sdAb comprises an amino acid sequence according to SEQ ID No. 2, an amino acid sequence with at least 90% sequence similarity with SEQ ID No. 2 or an amino acid sequence with at least 90% sequence identity with SEQ ID No. 2.

In embodiments, the sdAb comprises:
CDR1 with SEQ ID No. 14, CDR2 with SEQ ID No. 18 and CDR3 with SEQ ID No. 21,
CDR1 with SEQ ID No. 15, CDR2 with SEQ ID No. 19 and CDR3 with SEQ ID No. 22,
CDR1 with SEQ ID No. 16, CDR2 with SEQ ID No. 19 and CDR3 with SEQ ID No. 22,
CDR1 with SEQ ID No. 16, CDR2 with SEQ ID No. 19 and CDR3 with SEQ ID No. 23,
CDR1 with SEQ ID No. 16, CDR2 with SEQ ID No. 19 and CDR3 with SEQ ID No. 24, or
CDR1 with SEQ ID No. 17, CDR2 with SEQ ID No. 20 and CDR3 with SEQ ID No. 25.

In embodiments, the sdAb comprises: CDR1 with an amino acid sequence selected from the group comprising SEQ ID No. 14, SEQ ID No. 15 and SEQ ID No. 16, CDR2 with an amino acid sequence selected from the group comprising SEQ ID No. 18 and SEQ ID No. 19, and CDR3 with an amino acid sequence selected from the group comprising SEQ ID No. 21 to SEQ ID No. 24.

In embodiments, the sdAb comprises: CDR1 with an amino acid sequence according to SEQ ID No. 14, CDR2 with an amino acid sequence according to SEQ ID No. 18 and CDR3 with an amino acid sequence according to SEQ ID No. 21.

In embodiments, the sdAb has a size in the range of 11 kDa to 16 kDa, preferably in the range of 13 kDa to 15 kDa, more preferably in the range of 14.0 kDa to 14.5 kDa. Advantageously, a sdAb is smaller than an antibody. Surprisingly, good binding to CD7 could be demonstrated with the sdAb according to the invention despite its small size.

In embodiments, the sdAb has a sequence length in the range of 105 amino acids to 140 amino acids, preferably in the range of 110 amino acids to 130 amino acids.

In embodiments, the sdAb is in a multivalent form. In embodiments, the multivalent form of the sdAb is formed by bonding, chemically or by recombinant DNA techniques, of at least two sdAbs according to the invention. In embodiments, the sdAb is in a bivalent form (two bound sdAbs), a trivalent form (three bound sdAbs) or tetravalent form (four bound sdAbs). The sdAbs comprised within a multivalent construct may be identical or different.

In embodiments, the sdAb is associated with at least one of a detectable label, a chelator, a prosthetic group, a therapeutically and/or pharmacologically active agent.

In embodiments, the sdAb for use in the therapy of cancer, in particular theranostics, is associated with at least one therapeutically and/or pharmacologically active agent Advantageously, the sdAb for use in the therapy of cancer, in particular theranostics, targets therapeutically and/or pharmacologically active agent to the cancer cells.

As used herein, the term "detectable label" (also detectable marker) refers to a detectable compound or composition which is conjugated directly or indirectly associated with the sdAb, wherein it is detectable by itself (e. g., radioisotope labels or fluorescent labels) or, in the case of an enzymatic label, may catalyze chemical alteration of a substrate compound or composition which is detectable. The detectable label includes detectable moieties and tracers for immuno-histochemistry, optical imaging, near infrared imaging (NIR), positron emission tomography (PET), single photon emission computed tomography (SPECT) or magnetic resonance imaging (MRI).

In embodiments, the detectable label is selected from a tag, a dye, a fluorescent compound, a bioluminescent compound, a radioactive isotope, a contrast agent, an enzyme, a nanoparticle and/or a magnetic particle.

As used herein, the term "prosthetic group" refers to a non-polypeptide molecule required for the biological function of proteins. In embodiments, prosthetic groups are disclosed in Wester and Schottelius (Wester and Schottelius 2007). In embodiments, prosthetic groups are selected from SiFA (silicon-fluoride-acceptor), SFB (N-Succinimidyl-4-[¹⁸F]Fluorobenzoate), FBEM (¹⁸F-N-[2](4-Fluorobenzamido)ethyl]-maleimide), halides and sulfonates, in particular tosylates, triflates, nosylates and mesylates.

As used herein, the term "therapeutically and/or pharmacologically active agent" refers to a substance that inhibits or prevents a cellular function and/or causes cell death or destruction of a cancer cells, including radioactive isotopes, chemotherapeutic agents, enzymes and fragments thereof, such as nucleolytic enzymes; antibiotics, toxins, growth inhibitory agents and drug moieties.

As used herein, the term "chemotherapeutic agent" refers to a chemical compound useful in the treatment of cancer.

In embodiments, chemotherapeutic agents are selected from immunotoxins, chimeric antigen receptor T cells (CAR T cells) and switchable CAR T cells.

As used herein, the term "immunotoxin" refers to a protein comprising at least a targeting domain linked to a toxin. When the protein binds to that cell by the targeting domain, it is taken in through endocytosis, and the toxin kills the cell.

As used herein, the term "CAR" refers to an artificial receptor consisting of a binding moiety, which provides an antigen-specificity for a target cell and one or several signaling chains derived from immune receptors. Immune cells, genetically modified to express CARs, can be used to bind cells or tissue structures expressing the appropriate target of the CAR binding moiety. Cross-linking leads to an induction of signal pathways via the CAR signaling chains, which will change the biologic properties of the CAR-engrafted immune cell. Advantageously, CAR T immunotherapy is used to modify T cells to recognize cancer cells in order to effectively target and destroy them.

As used herein, the term "switchable CAR T cells" refer to T cells engineered to express a CAR with a binding domain recognizing a tag or being a tag. Antigen-specificity is provided by soluble targeting molecules, which consist of an antigen-binding domain fused to the tag or a tag-binding domain recognized by the switchable CAR.

In embodiments, chemotherapeutic agents are selected from the group comprising A-dmDT390-bisFv(UCHT1), bispecific CARs targeting CD19 and CD22; BL22, BM7PE, Cintredekin besudotox, Denileukin diftitox, DT2219, Hum-195/rGel, IgG-RFB4-SMPT-dgA, IL-4(38-37)-PE38KDEL, LMB-2, LMB-7, LMB-9, LMB-100, MOC31PE, Moxetumomab Pasudotox, MR1-1, RFT5pdgA, MT-3724, SS1(dsFv)-PE38, T-Guard, and Transferrin-CRM107.

In further embodiments, chemotherapeutic agents are selected from the group comprising alkylating agents, alkyl sulfonates, aziridines, ethylenimines and methylamelamines, acetogenins, delta-9-tetrahydrocannabinol, beta-lapachone, lapachol, colchicines, betulinic acid, camptothecin, bryostatin, callystatin, CC-1065, podophyllotoxin, podophyllinic acid, teniposide, cryptophycins, dolastatin, duocarmycin, eleutherobin, pancratistatin, a sarcodictyin, spongistatin, nitrogen mustards, nitrosoureas, antibiotics, dynemicin, an esperamicin, aclacinomysins, actinomycin, authramycin, azaserine, bleomycins, cactinomycin, carabicin, carminomycin, carzinophilin, chromomycins, dactinomycin, daunorubicin, detorubicin, 6-diazo-S-oxo-L-norleucine, ADRIAMYCIN^{™} doxorubicin, epirubicin, esorubicin, idarubicin, marcellomycin, mitomycins, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites, folic acid analogues, purine analogs, pyrimidine analogs, androgens, anti-adrenals, folic acid replenisher, aceglatone, aldophosphamide glycoside, aminolevulinic acid, eniluracil, amsacrine, bestrabucil, bisantrene, edatraxate, defofamine, demecolcine, diaziquone, elfomithine, elliptinium acetate, an epothilone, etoglucid, gallium nitrate, hydroxyurea, lentinan, lonidainine, maytansinoids, mitoguazone, mitoxantrone, mopidanmol, nitraerine, pentostatin, phenamet, pirarubicin, losoxantrone, 2-ethylhydrazide, procarbazine, PSK^{™} (Polysaccharide-K) polysaccharide complex, razoxane, rhizoxin, sizofiran, spirogermanium, tenuazonic acid, triaziquone, 2,2',2"-trichlorotriethylamine, trichothecenes, urethane, Vindesine (ELDISINE^{™}, FILDESIN^{™}), dacarbazine, mannomustine, mitobronitol, mitolactol, pipobroman, gacytosine, arabinoside ("Ara-C"), thiotepa, taxoids, chloranbucil, gemcitabine (GEMZAR^{™}), 6-thioguanine, mercaptopurine, methotrexate, platinum analogs, vinblastine (VELBAN^{™}), platinum, etoposide (VP-16), ifosfamide, mitoxantrone, Vincristine (ONCOVIN^{™}), oxaliplatin, leucovovin, Vinorelbine (NAVELBINE^{™}), novantrone, edatrexate, daunomycin, aminopterin, ibandronate, topoisomerase inhibitor RFS 2000, difluoromethylornithine (DMFO), retinoids, capecitabine (XELODA^{™}), pharmaceutically acceptable salts, acids or derivatives as well as combinations thereof.

In embodiments, chemotherapeutic agents are selected from the group comprising alkylating agents, such as thiotepa and CYTOXAN^{™} cyclosphosphamide; alkyl sulfonates, such as busulfan, improsulfan and piposulfan; aziridines, such as benzodopa, carboquone, meturedopa and uredopa; ethylenimines and methylamelamines, including altretamine, triethylenemelamine, triethylenephosphoramide, triethylenethiophosphoramide and trimethylolomelamine; acetogenins, especially bullatacin and bullatacinone; delta-9-tetrahydrocannabinol, especially dronabinol and MARINOL^{™}; beta-lapachone; lapachol; colchicines; betulinic acid; a camptothecin, including the synthetic analogue topotecan (HYCAMTIN^{™}), CPT-II (irinotecan, CAMPTOSAR^{™}), acetylcamptothecin, scopolectin, and 9-aminocamptothecin; bryostatin; callystatin; CC-1065, including its adozelesin, carzelesin and bizelesin synthetic analogues; podophyllotoxin; podophyllinic acid; teniposide; cryptophycins, particularly cryptophycin 1 and cryptophycin 8; dolastatin; duocarmycin, including the synthetic analogues, KW-2189 and CB I-TMI; eleutherobin; pancratistatin; a sarcodictyin; spongistatin; nitrogen mustards, such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosoureas, such as carmustine, chlorozotocin, fotemustine, lomustine, nimustine, and ranimnustine; antibiotics such as the enediyne antibiotics, e. g., calicheamicin, especially calicheamicin gammall and calicheamicin omegall; dynemicin, including dynemicin A; an esperamicin; as well as neocarzinostatin chromophore and related chromoprotein enediyne antibiotic chromophores, aclacinomysins, actinomycin, authramycin, azaserine, bleomycins, cactinomycin, carabicin, carminomycin, carzinophilin, chromomycins, dactinomycin, daunorubicin, detorubicin, Erdiazo-S-oxo-L-norleucine, ADRIAMYCIN^{™} doxorubicin, including morpholino-doxorubicin, cyanomorpholino-doxorubicin, 2-pyrrolino-doxorubicin and deoxydoxorubicin; epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, catmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK^{™} (Polysaccharide-K) polysaccharide complex; razoxane; rhizoxin; sizofiran; spirogermanium; tenuazonic acid; triaziquone; 2,2',2"-trichlorotriethylamine; trichothecenes, especially T-2 toxin, verracurin A, roridin A and anguidine; urethan; Vindesine (ELDISINE^{™}, FILDESIN^{™}); dacarbazine; mannomustine; mitobronitol; mitolactol; pipobroman; gacytosine; arabinoside ("Ara-C"); thiotepa; taxoids, e.g., TAXOL^{™}, paclitaxel, ABRAXANE^{™}, and TAXOTERET"" docetaxel; chlorambucil; gemcitabine (GEMZAR^{™}); 6-thioguanine; mercaptopurine; methotrexate; platinum analogs such as cisplatin and carboplatin; vinblastine (VELBAN^{™}); platinum; etoposide (VP-16); ifosfamide; mitoxantrone; Vincristine (ONCOVIN^{™}); oxaliplatin; leucovovin; Vinorelbine (NAVELBINE^{™}); novantrone; edatrexate; daunomycin; aminopterin; ibandronate; topoisomerase inhibitor RFS 2000; difluoromethylornithine (DMFO); retinoids such as retinoic acid; capecitabine (XELODA^{™}); pharmaceutically acceptable salts, acids or derivatives thereof; as well as combinations thereof.

In preferred embodiments, chemotherapeutic agents are selected from the group comprising thiotepa and CYTOXAN^{™} cyclosphosphamide, busulfan, improsulfan, piposulfan, benzodopa, carboquone, meturedopa, uredopa, ethylenimines, altretamine, triethylenemelamine, triethylenephosphoramide, triethylenethiophosphoramide, trimethylolomelamine, bullatacin, bullatacinone, dronabinol, MARINOL^{™}, beta-lapachone, lapachol, colchicines, betulinic acid, topotecan (HYCAMTIN^{™}), CPT-II (irinotecan, CAMPTOSAR^{™}), acetylcamptothecin, scopolectin, 9-aminocamptothecin, bryostatin, callystatin, CC-1065, CC-1065 adozelesin analogue, CC-1065 carzelesin analogue, CC-1065 bizelesin analogue, podophyllotoxin, podophyllinic acid, teniposide, cryptophycin 1, cryptophycin 8, dolastatin, duocarmycin, KW-2189, CB I-TMI; eleutherobin, pancratistatin, a sarcodictyin, spongistatin, chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard, carmustine, chlorozotocin, fotemustine, lomustine, nimustine, ranimnustine, enediyne antibiotics, calicheamicin, dynemicin, an esperamicin, neocarzinostatin chromophore, aclacinomysins, actinomycin, authramycin, azaserine, bleomycins, cactinomycin, carabicin, carminomycin, carzinophilin, chromomycins, dactinomycin, daunorubicin, detorubicin, 6-diazo-S-oxo-L-norleucine, ADRIAMYCIN^{™} doxorubicin, morpholino-doxorubicin, cyanomorpholino-doxorubicin, 2-pyrrolino-doxorubicin, deoxydoxorubicin, epirubicin, esorubicin, idarubicin, marcellomycin, mitomycins, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin, methotrexate, 5-fluorouracil (5-FU), denopterin, methotrexate, pteropterin, trimetrexate, fludarabine, 6-mercaptopurine, thiamiprine, thioguanine, ancitabine, azacitidine, 6-azauridine, catmofur, cytarabine, dideoxyuridine, doxifluridine, enocitabine, floxuridine, calusterone, dromostanolone propionate, epitiostanol, mepitiostane, testolactone, aminoglutethimide, mitotane, trilostane, frolinic acid, aceglatone, aldophosphamide glycoside, aminolevulinic acid, eniluracil, amsacrine, bestrabucil, bisantrene, edatraxate, defofamine, demecolcine, diaziquone, elfomithine, elliptinium acetate, an epothilone, etoglucid, gallium nitrate, hydroxyurea, lentinan, lonidainine, maytansine, ansamitocins, mitoguazone, mitoxantrone, mopidanmol, nitraerine, pentostatin, phenamet, pirarubicin, losoxantrone, 2-ethylhydrazide, procarbazine, PSK^{™} (Polysaccharide-K) polysaccharide complex, razoxane, rhizoxin, sizofiran, spirogermanium, tenuazonic acid, triaziquone, 2,2',2"-trichlorotriethylamine, T-2 toxin, verracurin A, roridin A, anguidine, urethane, Vindesine (ELDISINE^{™}, FILDESIN^{™}), dacarbazine, mannomustine, mitobronitol, mitolactol, pipobroman, gacytosine, arabinoside ("Ara-C"), thiotepa, TAXOL^{™}, paclitaxel, ABRAXANE^{™}, TAXOTERET^{M} docetaxel, chloranbucil, gemcitabine (GEMZAR^{™}), 6-thioguanine, mercaptopurine, methotrexate, cisplatin, carboplatin, vinblastine (VELBAN^{™}), platinum, etoposide (VP-16), ifosfamide, mitoxantrone, Vincristine (ONCOVIN^{™}), oxaliplatin, leucovovin, Vinorelbine (NAVELBINE^{™}), novantrone, edatrexate, daunomycin, aminopterin, ibandronate, topoisomerase inhibitor RFS 2000, difluoromethylornithine (DMFO), retinoic acid, capecitabine (XELODA^{™}), pharmaceutically acceptable salts or acids thereof; as well as combinations thereof.

As used herein, the term "toxin" refers to a naturally occurring organic substance having a detrimental effect on the growth or proliferation of a cell, produced by metabolic activities of living cells or organisms, in particular of bacterial, fungal, plant or animal origin. In embodiments, the toxin is a small molecule, peptide or protein. In embodiments, toxins are selected from exotoxins or endotoxins.

In embodiments, toxins are selected from the group comprising abrin, auristatins, such as auristatin E, auristatin F, monomethyl auristatin E (MMAE) and monomethyl auristatin F (MMAF); calicheamicins, cholera toxin, chlortetracycline, diphtheria toxin, daunorubicin, duocarmycins, dolastatins, liposome-encapsulated doxorubicin, maytansinoids, such as maytansinoid DM1 and DM4; maitansine, mitomycin, paclitaxel, pseudomonas exotoxin, ricin, ricin A and vinblastine.

In embodiments, the detectable label, therapeutically or pharmacologically active agent is an radioactive isotope selected from the group comprising ⁶⁸Ga, ¹⁸F, ⁸⁹Zr, ^{99m}Tc, ⁶¹Cu, ⁶⁴Cu, ⁶⁷Cu, ⁴³Sc, ⁴⁴Sc, ⁴⁷Sc, ¹⁴⁹Tb, ¹⁵²Tb, ¹⁵⁵Tb, ¹⁶¹Tb, ¹²³I, ¹²⁴I, ¹⁷⁷Lu, ²²⁵Ac, ²¹³Bi, ²¹¹At, ²¹²Pb and ²²³Ra, preferably ⁶⁸Ga.

In preferred embodiments, the sdAb is associated with at least one chelator and/or a prosthetic group and at least one radionuclide, in particular⁶⁸Ga, ¹⁸F, ⁸⁹Zr, ^{99m}Tc, ⁶¹Cu, ⁶⁴Cu, ⁶⁷Cu, ⁴³Sc, ⁴⁴Sc, ⁴⁷Sc, ¹⁴⁹Tb, ¹⁵²Tb, ¹⁵⁵Tb, ¹⁶¹Tb, ¹²³I, ¹²⁴I, ¹⁷⁷Lu, ²²⁵Ac, ²¹³Bi, ²¹¹At, ²¹²Pb and ²²³Ra, preferably ⁶⁸Ga.

A further aspect of the invention provides a pharmaceutical composition for use in the diagnosis and/or therapy of cancer in vivo comprising at least one of the sdAbs according to the invention.

The pharmaceutical composition is preferably administered parenterally, particularly preferred intravenously. In embodiments, the pharmaceutical composition is present in a form suitable for intravenous administration. Preferably, the pharmaceutical composition is a solution, emulsion or suspension.

In embodiments, the pharmaceutical composition comprises a pharmaceutically acceptable thinner (dilution agent) or carrier. In embodiments, the carrier is selected from water, an aqueous buffer solution, 0.9 % saline solution, 5 % glucose, 5 % xylitol, 0.3 % glycine solution, ringer solutions or amino acid solutions. In further embodiments, the aqueous buffer solution is selected from an aqueous histidine, sodium succinate, sodium acetate, sodium citrate, sodium phosphate or potassium phosphate-buffered solution with a pH value in the range of pH 5.0 to pH 7.0. In embodiments, the aqueous buffer solution has a buffer concentration in the range of 1 mmol/l (mM) to 500 mM, preferably in the range of 5 mM to 20 mM, especially preferred in the range of 5 mM to 10 mM.

In embodiments, the carrier comprises sodium chloride, preferably in a concentration in the range of 1 mM to 300 mM, especially preferred 150 mM.

In embodiments, the pharmaceutical composition further comprises a stabilizer, preferably with a concentration in the range of 1 mM to 900 mM, especially preferred in the range of 50 mM and 600 mM. In embodiments, the stabilizer is sucrose, trehalose or L-methionine.

In embodiments, the pharmaceutical composition further comprises a radical scavenger, in particular an antioxidant, preferably with a concentration in the range of 1 mM to 900 mM, especially preferred in the range of 20 mM and 200 mM. In embodiments, the radical scavenger is ascorbic acid ((5R)-[(1S)-1,2-Dihydroxyethyl]-3,4-dihydroxyfuran-2(5H)-one, Vitamin C) or gentisic acid (2,5-Dihydroxybenzoic acid). Advantageously, a radical scavenger, in particular an antioxidant, can react with and neutralize free radicals formed by radioactivity, which are highly reactive molecules that can initiate oxidation reactions. Thus, a radical scavenger prevents or delays the oxidative degradation of sensitive substances, in particular the sdAbs according to the invention.

In some embodiments, the pharmaceutical composition further comprises pharmaceutically acceptable excipients. The term "pharmaceutically acceptable excipients" refers to compounds, which provide approximately physiological conditions and/or increase the stability, such as agents for adjusting the pH value and buffering agents, agents for adjusting the toxicity and the like. In embodiments, pharmaceutically acceptable excipients are selected from sodium acetate, sodium chloride, potassium chloride, calcium chloride, sodium lactate and polysorbate-80, preferably polysorbate-80 in the range of 0.0001 % (w/v) to 1 % (w/v), especially preferred in the range of 0.001 % (w/v) to 0.1 % (w/v).

In preferred embodiments, the pharmaceutical composition comprises the sdAb in a dosage quantity in the range of 0.01 mg/day to 1.0 mg/day, preferably dosage quantities in the range of 0.01 mg/day to 0.1 mg/day.

In further embodiments, the pharmaceutical composition is sterile. The pharmaceutical composition is sterilized by conventional well-known techniques including, but not limited to, sterile filtration.

In embodiments, the pharmaceutical composition is used for administration to a subject.

In embodiments, the pharmaceutical composition is lyophilized prior to storage or stored as solution at ambient temperature or below, including, but not limited to, frozen storage.

In embodiments, the pharmaceutical composition is reconstituted and/or diluted in an infusion and stabilizer solution prior to administration to a subject. The solutions used for reconstitution or infusion/stabilization may contain any of the components mentioned for the pharmaceutical composition or similar components.

Another aspect of the invention is a kit for use in the diagnosis and/or therapy of cancer in vivo comprising
i. at least one sdAb according to the invention and
ii. at least one pharmaceutically acceptable excipient or a solution thereof.

In embodiments of the kit, the at least one sdAb is associated with at least one chelator and/or a prosthetic group.

The at least one pharmaceutically acceptable excipient or a solution thereof is used for reconstitution and/or dilution of the at least one sdAb prior to administration to a subject.

In embodiments, the at least one sdAb and/or the at least one pharmaceutically acceptable excipient or a solution thereof is sterile.

In embodiments, the at least one pharmaceutically acceptable excipient or a solution thereof is selected from sodium acetate, sodium chloride, potassium chloride, calcium chloride, sodium lactate and polysorbate-80, preferably sodium acetate.

In embodiments, the solution of the at least one pharmaceutically acceptable excipient has a concentration in the range of 0.0001 % (w/v) to 1 % (w/v), especially preferred in the range of 0.001 % (w/v) to 0.1 % (w/v).

In embodiments, the kit further comprises an inorganic acid. Advantageously, the inorganic acid is used for preparing a radioactive isotope, in particular for elution from a generator for a radioactive isotope.

In embodiments, the inorganic acid is hydrochloric acid.

In embodiments, the radioactive isotope is ⁶⁸Ga and the generator is a Ga/Ge generator.

In embodiments, the kit is used for the preparation of a sdAb associated with a radioactive isotope for the use in the diagnosis and/or therapy of cancer. Advantageously, the kit can be used to prepare the diagnostic and/or therapeutic agent immediately before use.

Another aspect of the invention is the use of sdAb, the pharmaceutical composition and/or the kit according to the invention in the diagnosis and/or therapy of cancer in vitro or ex vivo or for the synthesis of radioactive tracers and/or contrast agents.

A further aspect of the invention is a sdAb comprising an amino acid sequence according to one of the sequences selected from SEQ ID No. 2 to SEQ ID No. 13.

In embodiments, the sdAb comprises an amino acid sequence with at least 90% sequence similarity with one of the sequences selected from SEQ ID No. 2 to SEQ ID No. 13, preferably an amino acid sequence with at least 95% sequence similarity with one of the sequences selected from SEQ ID No. 2 to SEQ ID No. 13, most preferably an amino acid sequence with at least 99% sequence similarity with one of the sequences selected from SEQ ID No. 2 to SEQ ID No. 13.

In embodiments, the sdAb comprises an amino acid sequence with at least 90%, preferably at least 95%, more preferably at least 99%; sequence identity with one of the sequences selected from SEQ ID No. 2 to SEQ ID No. 13.

In embodiments, the sdAb comprises complementarity determining regions (CDR): CDR1 with an amino acid sequence selected from the group comprising SEQ ID No. 14 to SEQ ID No. 17, CDR2 with an amino acid sequence selected from the group comprising SEQ ID No. 18 to SEQ ID No. 20 and CDR3 with an amino acid sequence selected from the group comprising SEQ ID No. 21 to SEQ ID No. 25.

In embodiments, the sdAb comprises an amino acid sequence according to one of the sequences selected from SEQ ID No. 2, SEQ ID No. 4, SEQ ID No. 7, SEQ ID No. 8, SEQ ID No. 9 and SEQ ID No. 13, preferably an amino acid sequence according to one of the sequences selected from SEQ ID No. 2, SEQ ID No. 4, SEQ ID No. 7, SEQ ID No. 8 and SEQ ID No. 9.

In embodiments, the sdAb comprises an amino acid sequence according to one of the sequences selected from SEQ ID No. 2, SEQ ID No. 6, SEQ ID No. 10 and SEQ ID No. 11, preferably an amino acid sequence according to one of the sequences selected from SEQ ID No. 2, SEQ ID No. 10 and SEQ ID No. 11.

In embodiments, the sdAb comprises an amino acid sequence according to SEQ ID No. 2, an amino acid sequence with at least 90% sequence similarity with SEQ ID No. 2 or an amino acid sequence with at least 90% sequence identity with SEQ ID No. 2.

In embodiments, the sdAb comprises:
CDR1 with SEQ ID No. 14, CDR2 with SEQ ID No. 18 and CDR3 with SEQ ID No. 21,
CDR1 with SEQ ID No. 15, CDR2 with SEQ ID No. 19 and CDR3 with SEQ ID No. 22,
CDR1 with SEQ ID No. 16, CDR2 with SEQ ID No. 19 and CDR3 with SEQ ID No. 22,
CDR1 with SEQ ID No. 16, CDR2 with SEQ ID No. 19 and CDR3 with SEQ ID No. 23,
CDR1 with SEQ ID No. 16, CDR2 with SEQ ID No. 19 and CDR3 with SEQ ID No. 24, or
CDR1 with SEQ ID No. 17, CDR2 with SEQ ID No. 20 and CDR3 with SEQ ID No. 25.

In embodiments, the sdAb comprises: CDR1 with an amino acid sequence selected from the group comprising SEQ ID No. 14, SEQ ID No. 15 and SEQ ID No. 16, CDR2 with an amino acid sequence selected from the group comprising SEQ ID No. 18 and SEQ ID No. 19, and CDR3 with an amino acid sequence selected from the group comprising SEQ ID No. 21 to SEQ ID No. 24.

In embodiments, the sdAb comprises: CDR1 with an amino acid sequence according to SEQ ID No. 14, CDR2 with an amino acid sequence according to SEQ ID No. 18 and CDR3 with an amino acid sequence according to SEQ ID No. 21.

In embodiments, the sdAb has a size in the range of 11 kDa to 16 kDa, preferably in the range of 13 kDa to 15 kDa, more preferably in the range of 14.0 kDa to 14.5 kDa.

In embodiments, the sdAb has a sequence length in the range of 105 amino acids to 140 amino acids, preferably in the range of 110 amino acids to 130 amino acids.

In embodiments, the sdAb is in a multivalent form. In embodiments, the multivalent form of the sdAb is formed by bonding, chemically or by recombinant DNA techniques, of at least two sdAbs according to the invention. In embodiments, the sdAb is in a bivalent form (two bound sdAbs), a trivalent form (three bound sdAbs) or tetravalent form (four bound sdAbs). The sdAbs comprised within a multivalent construct may be identical or different.

In embodiments, the sdAb is associated with at least one of a detectable label, a chelator, a prosthetic group, a therapeutically and/or pharmacologically active agent.

In embodiments, the sdAb according to the invention is used for the ex vivo or in vitro detection of CD7 in a sample.

In embodiments, the sdAb according to the invention is used for the detection of immune cells, in particular T cells and NK cells. Advantageously, the distribution of immune cells, in particular T cells, can be detected in the human body to assess the activity of inflammatory process in cancer, infection, and chronic inflammatory diseases.

In embodiments, the sdAb according to the invention is used in the diagnosis of cancer. In embodiments, the sdAb according to the invention is used for the diagnosis of cancer by the direct imaging of immune cells, in particular T cells or NK cells, in immunotherapies.

In embodiments, the diagnosis is carried out before, during and/or after immunotherapy of cancer. In embodiments, the sdAb is used as radioactive tracer and/or contrast agent in non-invasive medical imaging of T cells in vivo, preferably by PET or SPECT imaging; or for the synthesis of radioactive tracers and/or contrast agents.

In embodiments, the sdAb according to the invention is used for the therapy of cancer or an autoimmune disease.

In further embodiments, the sdAb according to the invention is used for theranostics, i.e. the combined diagnosis and therapy, of immune cell related cancer, preferably lymphomas, in particular T cell or NK lymphomas; or leukaemia.

In embodiments, the sdAb according to the invention is used for the manufacture of a medicament for the treatment of cancer or an autoimmune disease.

A further aspect of the invention provides a nucleic acid comprising a nucleic acid sequence encoding for the sdAb according to the invention.

A further aspect of the invention provides a pharmaceutical composition comprising at least one sdAb according to the invention.

Another aspect of the invention is a method of diagnosing a subject in need thereof comprising administering a diagnostically effective dose of a sdAb according to the invention to the subject in need thereof, preferably a human having cancer.

Another aspect of the invention is a method of treating a subject in need thereof comprising administering a therapeutically effective dose of a sdAb according to the invention to the subject in need thereof, preferably a human, having cancer or an autoimmune disease.

The terms "subject," "patient," and "individual" interchangeably refer to an entity that is being diagnosed and/or treated. This can include, for example, a mammal, in particular a human or a non-human primate mammal. The mammal can also be a laboratory mammal, e.g., mouse, rat, rabbit, hamster. In some embodiments, the mammal can be an agricultural mammal (e.g., equine, ovine, bovine, porcine, camelid) or domestic mammal (e.g., canine, feline).

The term "diagnostically effective dose" or "therapeutically effective dose" refers to an amount sufficient to achieve the desired result. In embodiments, a diagnostically effective dose or therapeutically effective dose do not induce or cause undesirable side effects. A diagnostically effective dose or therapeutically effective dose can be determined by first administering a low dose, and then incrementally increasing that dose until the desired effect is achieved.

For diagnostic or therapeutic applications, a sterile pharmaceutical composition according to the invention or a sterile kit according to the invention, comprising a sdAb according to the invention is administered to a subject in order to diagnose or treat the illnesses.

In embodiments, the pharmaceutical composition is administered parenterally, preferably intravenously.

In embodiments of the method of diagnosing, the diagnosis is carried out after the administration of the sdAb according to the invention, preferably in the range of 10 min to 240 min after the administration of the sdAb according to the invention.

In embodiments of the method of diagnosing, the diagnosis is carried out by non-invasive medical imaging of T cells in vivo, preferably by NIR, PET, PET/CT, PET/MR, SPECT or MR imaging, more preferably by PET, PET/CT, PET/MR or SPECT imaging.

In embodiments of the method of treating, the sdAb according to the invention is administered as at least one dosage, preferably as at least two dosages.

In embodiments of the method of treating, the sdAb according to the invention is administered as at least two dosages, wherein the period between the two dosages is in the range of 6 weeks to 12 weeks.

In embodiments of the method of treating, the sdAb according to the invention is administered in combination with at least one further therapeutically and/or pharmacologically active agent and/or with at least one further therapy, in particular surgical removal of a tumor, radiotherapy, chemotherapy, stem cell transplantation, hyperthermia treatment and/or immunotherapy.

The invention is not limited to the embodiments shown and described, but also includes all embodiments having the same effect within the meaning of the invention. Furthermore, the invention is also not limited to the specifically described combinations of features but may also be defined by any other combination of specific features of all the individual features disclosed as a whole, provided that the individual features are not mutually exclusive, or a specific combination of individual features is not explicitly excluded.

In the following, the invention will be explained in more detail by means of an embodiment example. The example is intended to describe the invention without limiting it. Implementations of the invention will be described, by way of example only, with reference to accompanyingfigures in which:

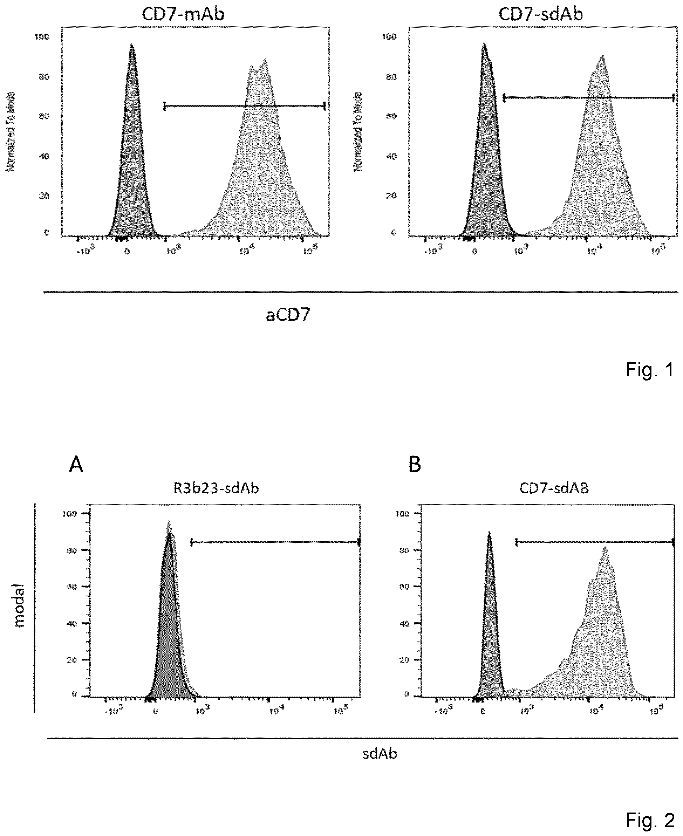

- **Fig. 1**: shows the analysis of the capability of the anti-CD7 single-domain antibody (CD7-sdAb) to bind human CD8+ T cells. Flow cytometry-based determination of binding specificity to human CD8+ T cells of CD7-sdAb. A non-targeting irrelevant sdAb (R3b23-sdAb) was used as a control to exclude unspecific binding of the sdAb basic framework-structure (first graph). A monoclonal antibody against CD7 was used as a positive control to verify possible binding of respective target antigens.
- **Fig. 2**: shows the analysis of anti-CD7 sdAb binding to retrovirally transduced U698M cells. Flow cytometry-based determination of binding specificity to retrovirally transduced B cell lymphoma tumor cell line U698M with CD7 target antigen. (A) Exemplary binding of R3b23 to CD7-transduced U698M cells, with wild type U698M used as a negative control. (B) CD7-sdAb binding to CD7-transduced U698M cells, with wild type U698M used as a negative control.

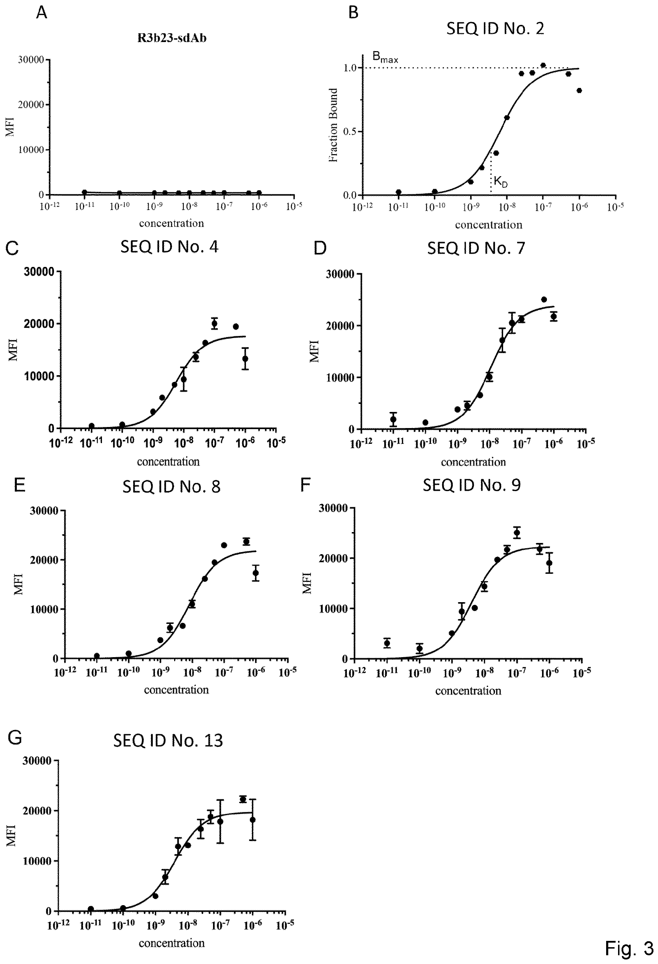

- **Fig. 3**: shows binding affinity of anti-CD7 sdAb on human CD8+ T cells. Determined affinity binding curves for CD7-sdAb (B-G) at different concentrations on human CD8+ T cells. R3b23-sdAb served as a negative control for unspecific binding (A). Binding ofCD7-sdAb according to SEQ ID No. 2 (B) is shown as a fraction of the maximum specific binding (Bₘₐₓ) and the binding intensity of R3b23-sdAb (A) and CD7-sdAbs according to SEQ ID No. 4, SEQ ID No. 7, SEQ ID No. 8, SEQ ID No. 9 and SEQ ID No. 13 are shown via the MFI at the different concentrations. Measurements were performed in triplicates and data is depicted on a semi-logarithmic scale. The dissociation constants (K_{D}) for CD7 were calculated based of one-site specific binding and are in the range of 3.90 nM to 11.0 nM.

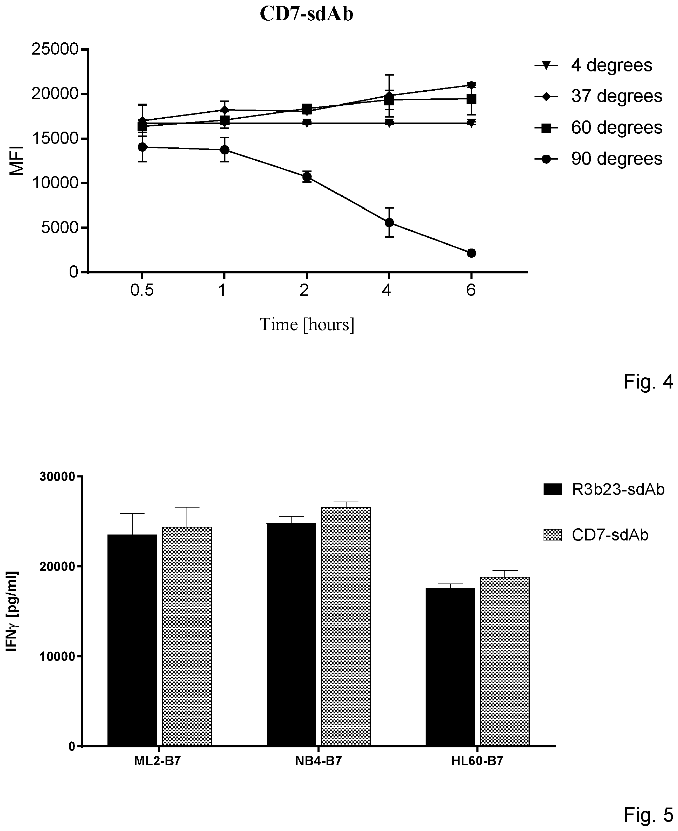

- **Fig. 4**: shows the analysis of the thermostability of the ability of the anti-CD7 sdAb to bind to human CD8+ T cells via flow cytometry analysis. CD7-sdAb kept at 4 °C, 37 °C, 60 °C or 90 °C for 0.5 h, 1 h, 2 h, 4 h or 6 h, after which their binding capabilities to human CD8+ T cells were analyzed via flow cytometry. The temperatures of sdAb were kept steady at 37 °C, 60 °C and 90 °C and 4° C. Measurements were performed in triplicates and flow cytometry data is depicted as MFI.
- **Fig. 5**: shows the analysis of the cytokine secretion of activated T cells (CD8+ T cells) after co-incubation with the anti-CD7 sdAb according to the invention by ELISA. IFNy levels were determined by ELISA after 4 h co-culture of human CD8+ T cells, the respective target cell lines and CD7-sdAb and R3b23-sdAb as a control. Measurements were performed in triplicates.

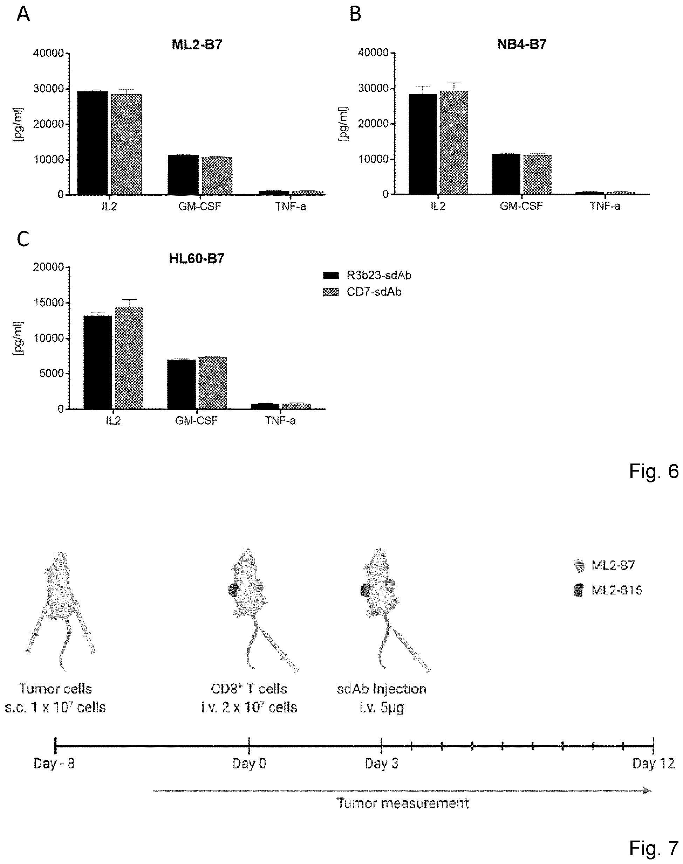

- **Fig. 6**: shows cytokine secretion analysis of IL2, GM-CSF and TNF-α by ELISA after co-incubation with CD7-sdAb. Cytokine secretion levels of IL-2, GM-CSF and TNF-α were determined by ELISA after 4 h co-culture of human CD8+ T cells, the respective target cell lines and CD7-sdAb and R3b23-sdAb as a control. Used target cell lines were ML2-B7 (A), NB4-B7 (B) and HL60-B7 (C). Measurements were performed in triplicates.
- **Fig. 7**: shows an in vivo analysis of T cell functionality of T cells after injection of the sdAb according to the invention. Schematic of the experimental setup of in vivo tumor rejection model for CD8+ T cells. NSG mice were subcutaneously (s.c.) injected with ML2-B7 cells in the right flank and ML2-B15 cells in the left flank. After eight days, TCR-transgenic human CD8+ T cells were injected intravenously (i.v.) through the tail vein, followed three days later by i.v. injection of R3-b23-sdAb, OKT11, RPA, or CD7-sdAb. Tumor growth was monitored from tumor onset until the end of experiment at day twelve.

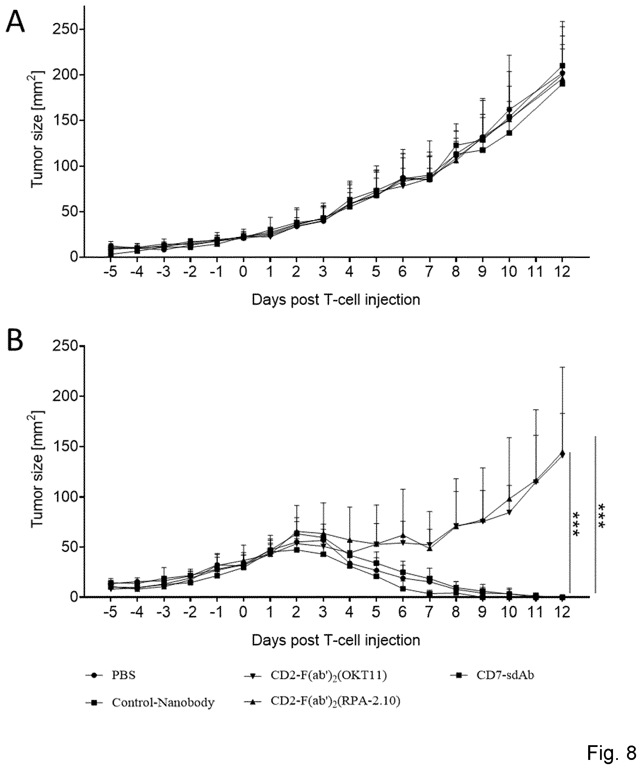

- **Fig. 8**: shows monitoring of tumor growth kinetics of ML2-B15 (A) and ML2-B7 tumors (B) in NSG mice. On day 0, eight days after subcutaneous tumor injection, mice were intravenously injected with TCR 2.5D6-transgenic CD8+ T cells and three days later with either PBS, R3b23-sdAb, CD2-F(ab')₂ (OKT11), CD2-F(ab')₂ (RPA-2.10) or CD7-sdAb. Kinetics of tumor growth were monitored daily for twelve days post T-cell injection. The experiment was ended at day twelve, at which point all relevant non-control tumors had been fully rejected. Tumor sizes are shown in mm² and mean values and SDs are depicted for each group of mice. PBS n = 4, R3b23-sdAb n = 3, CD2-F(ab')₂ (OKT11) n = 6, CD2-F(ab')₂ (RPA-2.10) n = 4, CD7-sdAb n = 5. Significance was calculated using Mann-Whitney test (* p ≤ 0.05, ** p ≤ 0.01, *** p ≤ 0.001). The application of the CD7-sdAb did not impair T-cell cytotoxicity in vivo.

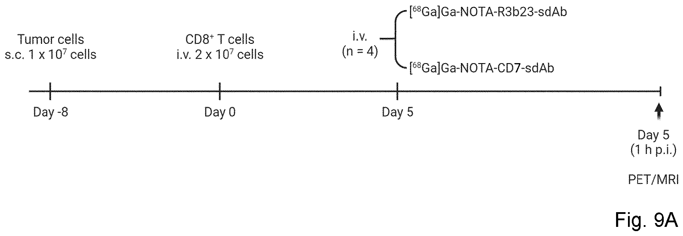

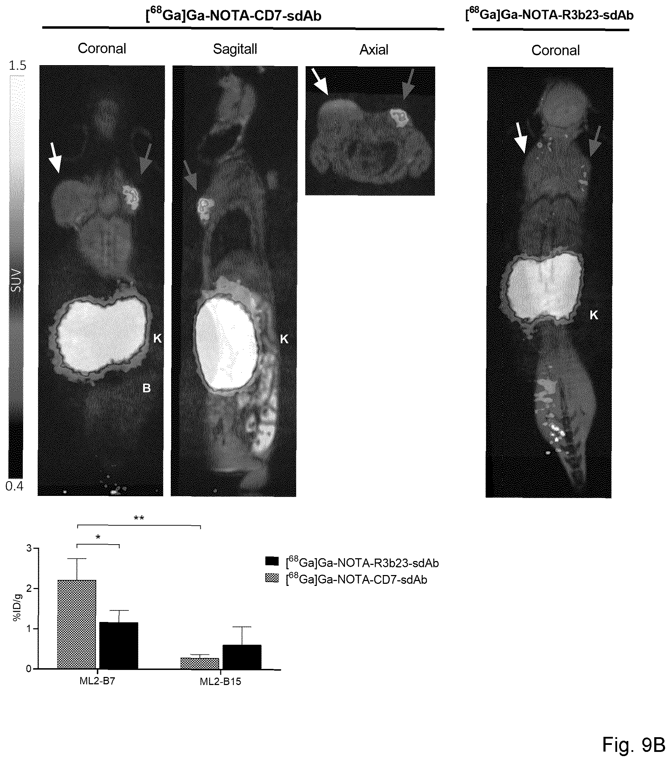

- **Fig. 9**: shows in vivo imaging using [⁶⁸Ga]Ga -NOTA-labeled anti-CD7 sdAb (CD7-sdAb) in NSG mice. **A)** Schematic of the experimental setup for in vivo imaging. Experimental layout of in vivo imaging study using intravenous injection of CD8+ T cells after ML2-B7 and -B15 tumor injection. Two groups of four mice per group were injected with each condition and 1 h p.i. scanned using PET/MRI. **B)** In vivo tracking of intravenously injected TCR-transgenic CD8+ T cells in ML2-B7 tumors using [⁶⁸Ga]Ga-NOTA-CD7-sdAb. PET/MRI images acquired 1 h p.i. of mice injected i.v. with [⁶⁸Ga]Ga-NOTA-CD7-sdAb (left) or [⁶⁸Ga]Ga-NOTA-R3b23-sdAb (right). Exemplary mice are shown in coronal, sagittal and axial orientation (A) or just in coronal orientation (B). 2·10⁷ TCR-transgenic CD8+ T cells were injected i.v. and the injected dose of applied tracer was 13 ±1 MBq per mouse. Scale bar is represented as percentage of injected dose per gram (%ID/g), 0.4 - 1.5 %ID/g. B = Bladder, K = Kidney. Striped arrow = ML2-B7 tumor, white arrow = ML2-B15 tumor. (below) Biodistribution data of [⁶⁸Ga]Ga-NOTA-CD7-sdAb and [⁶⁸Ga]Ga-NOTA-R3b23-sdAb in mice bearing ML2-B7 and -B15 tumors 1.5 h post injection and after previous PET/MR image acquisition. Mean %ID/g ± SD is depicted for each group of mice. [⁶⁸Ga]Ga-NOTA-R3b23-sdAb n = 4, [⁶⁸Ga]Ga-NOTA-CD7-sdAb n = 4. Significance was calculated using Mann-Whitney test (* p ≤ 0.05).

The functionality and specificity of the sdAb according to SEQ ID No. 2, SEQ ID No. 4, SEQ ID No. 7, SEQ ID No. 8, SEQ ID No. 9 and SEQ ID No. 13 was investigated, by examination of the binding to the T cells as well as to the target marker, CD7 (see **Fig. 1 to 3****).** For this purpose, the human tumor cell line U698M (B-cell lymphoma) was transduced with the specific gene encoding for CD7. Flow cytometry showed specific binding to the transduced cells compared to non-transduced controls. In addition, the strength of the binding affinity was determined for the sdAb by binding to human CD8+ T cells and it was 6.34·10⁻⁹ M (SEQ ID No. 2), 5.66·10⁻⁹ M (SEQ ID No. 4), 11.0·10⁻⁹ M (SEQ ID No. 7), 8.16·10⁻⁹ M (SEQ ID No. 8), 4.04·10⁻⁹ M (SEQ ID No. 9) and 3.90·10⁻⁹ M (SEQ ID No. 13), which is within an expected range. This demonstrates not only the functionality but also the specificity of the sdAb to the target.

Furthermore, the aspect of thermostability (see **Fig. 4****),** cytokine secretion (see **Fig. 5** **and** **6****),** functional impairment of cytotoxicity of T cells after co-incubation with the sdAb was investigated.

It was demonstrated that co-incubation had no negative effect on the cytotoxic potential of T cells, i.e., binding of the CD7 surface marker on T cells by the sdAb did not lead to impairment of their function in vitro to recognize and destroy tumor cells.

Regarding the binding of the anti-CD7 sdAb, no changes in IFNy secretions could be observed in all tumor cell lines examined (ML-2, NB-4 and HL-60) compared to the control group in which an irrelevant-binding sdAb (R3b23) was used. Thus, it can be suggested that the sdAb binding has no effect on the secretion of cytokines, which could trigger a cytokine storm syndrome in patients.

Furthermore, the in vivo effect of the CD2-binding F(ab')₂ fragments was compared to the CD7-sdAb (see **Fig. 7** **and** **8****).** CD2-binding F(ab')₂ fragments were used as positive controls as they impair the T-cell functionality. The group receiving the sdAb showed a picture congruent with the PBS control. The tumors were detected and rejected. Thus, it could be shown that the sdAb not only has no influence on the functionality of the T cells in vitro, but also has no effect on the function of the T cells in the mouse model in vivo. The use of CD2-binding F(ab')₂ fragments (positive control) in vivo showed four days after T cell injection a significant increase in tumor volume and thus, a functional impairment of T cells.

Based on the in vitro and in vivo data regarding purity, binding strength and specificity, as well as the lack of restriction of T cell functionality after sdAb binding, the potential of the CD7 sdAb for immuno-PET imaging was then investigated (see **Fig. 9****).** In this mouse experiment, four mice received [⁶⁸Ga]Ga-NOTA-R3b23-sdAb and were used for sdAb-based PET imaging and four mice were injected with the irrelevant sdAb [⁶⁸Ga]Ga-NOTA-R3b23-sdAb to serve as a control. For this purpose, ML-2 tumor cells were transduced with genes coding for HLA-B*07:02 (B7) or HLA-B*15:01 (B15) and injected into the left (B15) and right (B7) shoulders of the mice. Tumors bearing HLA-B15 served as negative controls. After successful growth of the tumor cells eight days after injection, human CD8+ T cells were intravenously injected through the tail vein. Five days later, the radiolabeled sdAbs were injected intravenously.

To allow imaging by PET imaging, the sdAb was previously conjugated with the p-SCN-Bn-NOTA chelator and labeled with the radioactive isotope gallium-68 (⁶⁸Ga) at room temperature, which with its short half-life of 67 minutes would be very advantageous for potential clinical application. This demonstrated that the sdAb targeting CD7 is capable of binding to T cells in vivo and imaging them by PET imaging.

In addition, the radioactive labeling of the sdAb was developed and optimized, which is essential for imaging by PET. The tracer was labeled with the radioactive isotope gallium-68 and then evaluated for its imaging properties. The result of PET imaging showed a clear accumulation in the treated experimental tumor ML2-B7 which was infiltrated by T cells already one hour after injection of the tracer. This underlines the rapid and specific accumulation of the tracer.

Furthermore, apart from an expected accumulation in the kidneys (due to excretion), no noticeable accumulation of the tracer in other organs or in the control tumor (ML2-B15) could be observed and therefore could be shown that the radiolabeled sdAb is able to bind to T cells in the mouse model without loss of function and to visualize them specifically and clearly by PET imaging.

### Cited non-patent literature

Auletta S, lodice V, Galli F, Lepareur N, Devillers A, Signore A (2018) Study of Binding Kinetics and Specificity of 99mTc-SSS-Complex and 99mTc-HMPAO to Blood Cells. Contrast Media Mol Imaging 5603902. doi: 10.1155/2018/5603902.
Dromain C, Beigelman C, Pozzessere C, Duran R, Digklia A (2020) Imaging of tumour response to immunotherapy. Eur Radiol Exp. 4(1):2. doi: 10.1186/s41747-019-0134-1.
Druker BJ, Guilhot F, O'Brien SG, Gathmann I, Kantarjian H, Gattermann N, Deininger MW, Silver RT, Goldman JM, Stone RM, Cervantes F, Hochhaus A, Powell BL, Gabrilove JL, Rousselot P, Reiffers J, Cornelissen JJ, Hughes T, Agis H, Fischer T, Verhoef G, Shepherd J, Saglio G, Gratwohl A, Nielsen JL, Radich JP, Simonsson B, Taylor K, Baccarani M, So C, Letvak L, Larson RA; IRIS Investigators (2006) Five-year follow-up of patients receiving imatinib for chronic myeloid leukemia. N Engl J Med. 355(23):2408-2417. doi: 10.1056/NEJMoa062867.
Käsbauer T, Gosmann D, Russelli L, Bassermann F, Weber W, D'Alessandria C, Krackhardt AM (2019) In vitro characterisation of nanobodies targeting a pan-T-cell marker as potential universal immunotherapy T-cell trackers, World Molecular Imaging Congress Montreal, 04.-07.09.2019.Lang D, Wahl G, Poier N, Graf S, Kiesl D, Lamprecht B, Gabriel M. (2020) Impact of PET/CT for Assessing Response to Immunotherapy-A Clinical Perspective. J Clin Med. 9(11):3483. doi: 10.3390/jcm9113483.
Tang J, Li J, Zhu X, Yu Y, Chen D, Yuan L, Gu Z, Zhang X, Qi L, Gong Z, Jiang P, Yu J, Meng H, An G, Zheng H, Yang L (2016) Novel CD7-specific nanobody-based immunotoxins potently enhanced apoptosis of CD7-positive malignant cells. Oncotarget. 7(23):34070-83. Weist MR, Starr R, Aguilar B, Chea J, Miles JK, Poku E, Gerdts E, Yang X, Priceman SJ, Forman SJ, Colcher D, Brown CE, Shively JE (2018) PET of Adoptively Transferred Chimeric Antigen Receptor T Cells with 89Zr-Oxine. J Nucl Med. 59(10):1531-1537. doi: 10.2967/jnumed.117.206714.
Wester HJ, Schottelius M (2007) Fluorine-18 Labeling of Peptides and Proteins. In: Schubiger PA, Lehmann L, Friebe M (eds) PET Chemistry. Ernst Schering Research Foundation Workshop 64. Springer. https://doi.org/10.1007/978-3-540-49527-7_4.

## Claims

1. A single domain antibody which binds to human CD7 comprising complementarity determining regions (CDR):
CDR1 with an amino acid sequence selected from the group comprising SEQ ID No. 14 to SEQ ID No. 17,
CDR2 with an amino acid sequence selected from the group comprising SEQ ID No. 18 to SEQ ID No. 20 and
CDR3 with an amino acid sequence selected from the group comprising SEQ ID No. 21 to SEQ ID No. 25,
for use in the diagnosis and/or therapy of cancer in vivo.

2. The single domain antibody for use in the diagnosis and/or therapy of cancer according to claim 1 comprising CDR1 with an amino acid sequence according to SEQ ID No. 14, CDR2 with an amino acid sequence according to SEQ ID No. 18 and CDR3 with an amino acid sequence according to SEQ ID No. 21.

3. The single domain antibody for use in the diagnosis and/or therapy of cancer according to claim 1 or 2 comprising an amino acid sequence according to one of the sequences selected from SEQ ID No. 2 to SEQ ID No. 13 or at least 90% sequence identity with one of the sequences selected from SEQ ID No. 2 to SEQ ID No. 13.

4. The single domain antibody for use in the diagnosis and/or therapy of cancer according to one of the claims 1 to 3, wherein the single domain antibody has a size in the range of 11 kDa to 16 kDa.

5. The single domain antibody for use in the diagnosis and/or therapy of cancer according to one of the claims 1 to 4, wherein the single domain antibody is associated with at least one of a detectable label, a chelator, a prosthetic group, a therapeutically and/or pharmacologically active agent.

6. The single domain antibody for use in the diagnosis and/or therapy of cancer according to claim 5, wherein the detectable label, therapeutically or pharmacologically active agent is a radioactive isotope selected from the group comprising ⁶⁸Ga, ¹⁸F, ⁸⁹Zr, ^{99m}Tc, ⁶¹Cu, ⁶⁴Cu, ⁶⁷Cu, ⁴³Sc, ⁴⁴Sc, ⁴⁷Sc, ¹⁴⁹Tb, ¹⁵²Tb, ¹⁵⁵Tb, ¹⁶¹Tb, ¹²³I, ¹²⁴I, ¹⁷⁷Lu, ²²⁵Ac, ²¹³Bi, ²¹¹At, ²¹²Pb and ²²³Ra.

7. The single domain antibody for use in the diagnosis and/or therapy of cancer according to one of the claims 1 to 6, wherein the single domain antibody is used as radioactive tracer and/or contrast agent in non-invasive medical imaging in vivo, preferably by PET or SPECT imaging.

8. A pharmaceutical composition comprising at least one of the single domain antibodies as defined in one of the claims 1 to 6 for use in the diagnosis and/or therapy of cancer in vivo.

9. A kit comprising
i. at least one single domain antibody as defined in one of the claims 1 to 6 and
ii. at least one pharmaceutically acceptable excipient or a solution thereof, for use in the diagnosis and/or therapy of cancer in vivo.

10. The kit for use in the diagnosis and/or therapy of cancer in vivo according to claim 9, wherein the at least one single domain antibody is associated with at least one chelator and/or a prosthetic group.

11. A single domain antibody which binds to human CD7 comprising an amino acid sequence according to one of the sequences selected from SEQ ID No. 2 to SEQ ID No. 13 or at least 90% sequence identity with one of the sequences selected from SEQ ID No. 2 to SEQ ID No. 13.

12. The single domain antibody of claim 11, wherein the single domain antibody is associated with at least one of a detectable label, a chelator, a prosthetic group, a therapeutically and/or pharmacologically active agent.

13. A nucleic acid comprising a nucleic acid sequence encoding for the single domain antibody according to claim 11 or 12.

14. A pharmaceutical composition comprising at least one single domain antibody according to claim 11 or 12.

15. Use of the single domain antibody as defined in one of the claims 1 to 6, the pharmaceutical composition as defined in claim 8 and/or the kit as defined in claim 9 or 10 in the diagnosis and/or therapy of cancer in vitro or ex vivo or for the synthesis of radioactive tracers and/or contrast agents.
